(19) Europäisches Patentamt

European Patent Office

Office européen des brevets

(11) Publication number: **0 070 665**
**B1**

(12) **EUROPEAN PATENT SPECIFICATION**

(45) Date of publication of patent specification: **11.09.85**

(51) Int. Cl.⁴: **C 07 C 46/08, C 07 C 50/04**

(21) Application number: **82303639.7**

(22) Date of filing: **12.07.82**

(54) **Process for oxidising phenol to P-benzoquinone.**

(30) Priority: **20.07.81 US 284893**

(43) Date of publication of application:
**26.01.83 Bulletin 83/04**

(45) Publication of the grant of the patent:
**11.09.85 Bulletin 85/37**

(84) Designated Contracting States:
**BE DE FR GB IT NL**

(56) References cited:
**EP-A-0 015 221**
**US-A-3 625 983**
**US-A-3 987 068**

(73) Proprietor: **Sun Refining and Marketing Company**
**1801 Market Street**
**Philadelphia, PA 19103 (US)**

(72) Inventor: **Hsu, Chao-Yang**
**615 Farnum Road**
**Media Pennsylvania 19063 (US)**
Inventor: **Lyons, James E.**
**612 Creekside Lane**
**Wallingford Pennsylvania 19086 (US)**

(74) Representative: **Lewin, John Harvey et al**
**Elkington and Fife High Holborn House 52/54**
**High Holborn**
**London WC1V 6SH (GB)**

Courier Press, Leamington Spa, England.

**Description**

This invention relates to a process for oxidising phenol to p-benzoquinone.

It is known in the art to oxidise phenol to p-benzoquinone with oxygen in the presence of a copper ion catalyst and such a process is disclosed in U.S. Patent No. 3,987,068. In that disclosure the oxidation is carried out in a nitrile solvent using a complex formed from the copper catalyst and the solvent and the operating conditions are said to be at temperatures of from about 0° to 100°C and a partial pressure of oxygen of from 7.1 to 203 bar, preferably 14.2 to 101 bar (about 7 to 200, preferably 14 to 100, atmospheres). As pointed out in U.S. Patent No. 3,987,068, the yield of quinone product increases with increased partial pressure of oxygen and it appears from the data therein to require partial pressures of oxygen above 101 bar (about 100 atmospheres) in order to achieve conversions of phenol to p-benzoquinone of the order of about 75%. Such pressures are too high to be useful in an economical commercial process because they require special equipment of high capital cost.

EP—A 15221 claims an improvement in the above process by carrying out the process in a ketone as solvent and also in the presence of carboxylate or phenate ions.

We have now found that the copper-catalysed process for oxidation of phenol to p-benzoquinone can be significantly improved so as to enable operation at lower, commercially useful pressures and still achieve an improved conversion and/or improved selectivity to product. In accordance with the invention, such objectives are achieved by conducting the oxidation of phenol in a nitrile and in the presence of a divalent copper ion catalyst (e.g. $Cu^{++}$) which is promoted with an alkali metal base wherein the molar ratio of the base to the copper catalyst is no greater than about 2.0.

Thus, the present invention provides a process for oxidising phenol to benzoquinone with a bivalent copper salt catalyst selected from the group of halides and nitrate, characterised in that increased selectivity is obtained by promoting the catalyst with an alkali metal base, the molar ratio of the base to the copper catalyst being no greater than about 2.0, and the process being carried out in a nitrile solvent.

In carrying out the process of the invention conventional temperature conditions, solvent systems and a choice of divalent (i.e., cupric) copper catalyst may be used. Thus, a temperature of from 20° to 100°C (preferably 50° to 75°C) and a nitrile solvent, preferably acetonitrile, are usually employed. The copper catalyst will be preferably a copper (II) halide, preferably chloride, although nitrate is operable and mixtures of such salts also may be used. However, other copper (II) salts such as acetates, sulphates, benzoates, carbonates, phosphates, and bisulphates have been found not to be effective catalysts for the reaction. We have also found that when a monovalent copper catalyst is used, the alkali metal base is not effective in enhancing the rate of reaction or selectivity to benzoquinone product. The alkali metal base promoter will be a Group I metal base such as lithium, sodium, potassium, cesium and rubidium and will be preferably a hydroxide such as lithium, sodium and potassium hydroxide, but carbonates and weak alkali metal bases such as acetates, phenolates, methylates and the like are also useful. As indicated above, the reaction can be carried out at moderate pressures and such pressures will generally be from 6.9 to 34.5 bar gauge (about 100 to 500 psig) partial pressure of oxygen, preferably from 13.8 to 27.6 bar gauge (about 200 to 400 psig). Mixtures of oxygen and nitrogen, air alone, or oxygen alone may be used, but preferably mixtures of oxygen and nitrogen such as air will be employed as the oxygenating medium.

It is of interest to note that the alkali metal promoters are not effective with monovalent copper ion (e.g. $Cu^+$) catalysts. We have also found that when the mole ratio of the base promoter to copper catalyst used exceeds about 2.0 the effect of the promoter is significantly reduced. The preferred ratio of promoter to copper is generally from 1.0 to 2.0.

A still further improvement in selectivity to benzoquinone is obtained when the oxidation method of this invention is carried out in the presence of water. The amount of water which imparts this improvement need not be large and, in fact, any amount less than about 10% by volume of the reaction solution is quite effective. More than about 10% should be avoided because conversion is lowered.

In order to further illustrate the invention, the following Examples are given:

Example 1

A solution of phenol in 5 ml. of acetonitrile which was agitated in a magnetically stirred mini-autoclave under an initial total pressure of 51.7 bar gauge (750 psig) was oxidised over a three hour period with a mixture of 40% (vol.) oxygen and 60% nitrogen in the presence of a $CuCl_2$ catalyst. The reaction parameters and results obtained are shown in the following Table I.

TABLE I

Effect of a group IA base on the copper (II) chloride catalyzed oxidation of phenol

| Catalyst | M moles cat | Base | M moles base | M moles phenol | Temp. (°C) | Time (min.) | % Selectivity to PBQ[1] | % Conversion to phenol | % Yield to PBQ[1] |
|---|---|---|---|---|---|---|---|---|---|
| $CuCl_2$ | 0.55 | — | — | 3.81 | 65 | 180 | 42 | 85 | 36 |
| $CuCl_2$ | 0.55 | LiOH | 0.55 | 7.36 | 65 | 180 | 50 | 98 | 49 |
| $CuCl_2$ | 0.55 | LiOH | 0.55 | 7.36 | 65 | 180 | 46 | 98 | 45 |
| $CuCl_2$ | 0.55 | LiOH | 0.55 | 7.36 | 65 | 180 | 48 | 98 | 47 |

[1]PBQ=p-Benzoquinone

The data in Table I clearly shows the formation of more PBQ in the presence of the alkali metal promoter than in its absence.

Example II

Following the experimental details of Example I with 0.55 mmoles of catalyst, additional runs were carried out. In some runs water was present during the oxidations. Table II shows the reaction parameters with numerous agents present and also shows the results obtained.

4

# 0 070 665

TABLE II
Oxidation of phenol to PBQ using Cu(II)

| Run No. | Promoter | Promoter (mmoles) | Modifier | Modifier (mmoles) | Phenol used (mmoles) | Conversion of phenol (%) | Selectivity to PBQ (%) |
|---|---|---|---|---|---|---|---|
| 1 | — | — | — | — | 4 | 83 | 39 |
| 2 | — | — | — | — | 8 | 73 | 49 |
| 3 | — | — | — | — | 12 | 32 | 51 |
| 4 | — | — | — | — | 16 | 32 | 55 |
| 5 | — | — | — | — | 20 | 23 | 62 |
| 6 | — | — | — | — | 34 | 37 | 16 |
| 7 | — | — | — | — | — | — | — |
| 8 | — | — | Water | 14.0 | 7.5 | 35 | 48 |
| 9 | LiCl | 0.55 | — | — | 7.5 | 99 | 52 |
| 10 | LiCl | 0.55 | Water | 14.0 | 7.5 | 87 | 54 |
| 11 | LiOH | 0.55 | — | — | 7.5 | 98 | 48 |
| 12 | LiOH | 0.55 | Water | 14.0 | 7.5 | 60 | 68 |
| 13 | LiOH | 1.10 | Water | 14.0 | 7.5 | 46 | 74 |
| 14 | LiOH | 1.65 | Water | 14.0 | 7.5 | 13 | 27 |
| 15 | KOH | 0.55 | Water | 14.0 | 7.5 | 58 | 61 |
| 16 | KOH | 1.10 | Water | 28.0 | 7.5 | 42 | 67 |
| 17 | LiOAc | 0.55 | — | — | 7.5 | 94 | 59 |
| 18 | NaOAc | 0.55 | — | — | 7.5 | 92 | 58 |
| 19 | KOAc | 0.55 | — | — | 7.5 | 83 | 51 |
| 20 | RbOAc | 0.55 | — | — | 7.5 | 66 | 65 |
| 21 | $Na_2CO_3$ | 0.55 | — | — | 7.5 | 97 | 55 |
| 22 | NaOPh | 0.55 | — | — | 7.5 | 98 | 55 |
| 23 | NaOPh | 1.10 | — | — | 7.5 | 68 | 57 |
| 24 | LiOPh | 0.55 | — | — | 7.5 | 95 | 49 |
| 25 | LiOPh | 1.10 | — | — | 7.5 | 92 | 49 |
| 26 | LiOMe | 0.55 | — | — | 7.5 | 98 | 50 |
| 27 | LiOMe | 0.55 | — | — | 7.5 | 94 | 37 |
| 28 | NaOMe | 0.55 | — | — | 7.5 | 95 | 52 |
| 29 | NaOMe | 1.10 | — | — | 7.5 | 79 | 37 |

# 0 070 665

As can be seen from the data in Table II, the presence of promoter and water contributes to improved selectivity. Of particular interest are runs 2, 8, 11, 12 and 13. Comparison of runs 2 and 8 show that the addition of water to a system without promoter actually reduces conversion. Comparison of runs 2 and 11 shows improved conversion with the alkaline promoter.

Comparison of runs 11 and 12 show that when promoter is present, the presence of water improves selectivity. It should be understood, of course, that an improvement in selectivity is of much greater significance than improved conversion since conversion can always be increased by recycling the unreacted reagents. Comparative runs 13 and 14 show that when the mole ratio of promoter to catalyst is higher than about 2.0, selectivity is reduced. Comparative runs 2, 9 and 10 show alkali metal halides to be relatively ineffective to increase selectivity without or with water. Table II also illustrates the use of alkali metal acetates, phenolates, carbonates and methylates as being effective to increase conversion of phenol in the process of the invention.

Example III

A solution of phenol in 5 ml. of acetonitrile containing the catalyst and promoter was oxidised with a mixture of 40% oxygen and 60% nitrogen in a glass-lined, magnetically stirred reactor. The reactor was heated to 65°C. and the reaction time was three hours. The following Table III gives the data and results obtained:

TABLE III

The effect of aqueous alkali on the selectivity of p-benzoquinone (PBQ) and on the quantity of chlorophenols formed during the copper-catalyzed oxidation of phenol

| Catalyst (mmols) | Base (mmols) | $H_2O$ (mmols) | Phenol conv. (%) | PBQ sel. (%) | Chlorophenol yield (%) |
|---|---|---|---|---|---|
| $CuCl_2$ (0.55) | —(0.0) | 0 | 65 | 44 | 3.1 |
| $CuCl_2$ (0.55) | —(0.0) | 7 | 50 | 52 | 3.9 |
| $CuCl_2$ (0.50) | NaOH (0.25) | 7 | 83 | 56 | 0.2 |
| $CuCl_2$ (0.50) | NaOH (0.50) | 14 | 35 | 71 | 1.1 |
| $CuCl_2$ (0.55) | KOH (0.55) | 14 | 58 | 61 | — |
| $CuCl_2$ (0.55) | LiOH (0.55) | 14 | · 60 | 68 | 1.3 |
| $CuCl_2$ (0.55) | LiOH (1.10) | 14 | 46 | 74 | — |

As can be seen from the above data another advantage of the process is the very low level of chlorophenol by-products formed when the aqueous alkali metal base promoter system is used.

**Claims**

1. A process for oxidising phenol to benzoquinone with a bivalent copper salt catalyst selected from the group of halides and nitrate, characterised in that increased selectivity is obtained by promoting the catalyst with an alkali metal base, the molar ratio of the base to the copper catalyst being no greater than about 2.0, and the process being carried out in a nitrile solvent.

2. A process as claimed in claim 1, wherein the solvent is acetonitrile.

3. A process for oxidising phenol to p-benzoquinone with a bivalent copper salt catalyst selected from the group of halides and nitrates in an acetonitrile solvent system, characterised in that increased selectivity is obtained by promoting the catalyst with an alkali metal base, the molar ratio of the base to the copper catalyst being no greater than about 2.0.

4. A process as claimed in any of claims 1 to 3, wherein the catalyst is cupric chloride.

5. A process as claimed in any of claims 2 to 4, wherein the process is carried out in the presence of water in an amount less than about 10% by volume of the reaction solution.

6. A process as claimed in any of claims 1 to 5, wherein the promoter is an alkali metal hydroxide.

7. A process as claimed in claim 6, wherein the promoter is lithium hydroxide.

8. A process as claimed in claim 6, wherein the promoter is potassium hydroxide.

9. A process as claimed in claim 6, wherein the promoter is sodium hydroxide.

10. A process as claimed in any of claims 1 to 9, wherein the molar ratio of the base to the copper catalyst is from 1.0 to 2.0.

**0 070 665**

**Patentansprüche**

1. Verfahren zur Oxidation von Phenol zu Benzochinon mit Hilfe eines zweiwertigen Kupfersalzes aus der Gruppe der Halogenide und Nitrate als Katalysator, dadurch gekennzeichnet, daß erhöhte Selektivität erreicht wird, indem eine Alkalimetallbase als Promotor für den Katalysator eingesetzt wird, wobei das Molverhältnis der Base zu dem Kupfer-Katalysator nicht mehr als etwa 2,0 beträgt, und das Verfahren in einem Nitril-Lösungsmittel durchgeführt wird.

2. Verfahren nach Anspruch 1, bei dem das Lösungsmittel Acetonitril ist.

3. Verfahren zur Oxidation von Phenol zu p-Benzochinon mit Hilfe eines zweiwertigen Kupfersalzes aus der Gruppe der Halogenide und Nitrate als Katalysator in einem Acetonitril-Lösungsmittelsystem, dadurch gekennzeichnet, daß erhöhte Selektivität erreicht wird, indem als Promotor für den Katalysator eine Alkalimetallbase eingesetzt wird, wobei das Molverhältnis der Base zu dem Kupferkatalysator nicht mehr als etwa 2,0 beträgt.

4. Verfahren nach einem der Ansprüche 1 bis 3, bei dem der Katalysator Kupfer-II-chlorid ist.

5. Verfahren nach einem der Ansprüche 2 bis 4, wobei das Verfahren in Gegenwart von Wasser in einer Menge von weniger als etwa 10 Vol.-% der Reaktionslösung durchgeführt wird.

6. Verfahren nach einem der Ansprüche 1 bis 5, bei dem der Promotor ein Alkalimetallhydroxid ist.

7. Verfahren nach Anspruch 6, bei dem der Promotor Lithiumhydroxid ist.

8. Verfahren nach Anspruch 6, bei dem der Promotor Kaliumhydroxid ist.

9. Verfahren nach Anspruch 6, bei dem der Promotor Natriumhydroxid ist.

10. Verfahren nach einem der Ansprüche 1 bis 9, bei dem das Molverhältnis der Base zu dem Kupfer-Katalysator 1,0 bis 2,0 beträgt.

**Revendications**

1. Procédé d'oxydation du phénol en benzoquinone avec un catalyseur à sel de cuivre bivalent, choisi dans le groupe constitué par les halogénures et le nitrate, caractérisé en ce qu'on obtient une sélectivité accrue en activant le catalyseur avec une base de métal alcalin, le rapport molaire de la base au catalyseur au cuivre ne dépassant pas 2 environ, et le procédé étant réalisé dans un solvant nitrile.

2. Procédé selon la revendication 1, dans lequel le solvant est l'acétonitrile.

3. Procédé d'oxydation du phénol en p-benzoquinone avec un catalyseur à sel de cuivre bivalent, choisi dans le groupe constitué par les halogénures et nitrates, dans un système solvant à l'acétonitrile, caractérisé en ce qu'on obtient une sélectivité accrue en activant le catalyseur avec une base de métal alcalin, le rapport molaire de la base au catalyseur au cuivre ne dépassant pas 2 environ.

4. Procédé selon une des revendications 1 à 3, dans lequel le catalyseur est le chlorure cuprique.

5. Procédé selon une des revendications 2 à 4, dans lequel on opère en présence d'eau en quantité inférieure à environ 10% du volume de la solution de réaction.

6. Procédé selon une des revendications 1 à 5, dans lequel l'activeur est un hydroxyde de métal alcalin.

7. Procédé selon la revendication 6, dans lequel l'activeur est l'hydroxyde de lithium.

8. Procédé selon la revendication 6, dans lequel l'activeur est l'hydroxyde de potassium.

9. Procédé selon la revendication 6, dans lequel l'activeur est l'hydroxyde de sodium.

10. Procédé selon une des revendications 1 à 9, dans lequel le rapport molaire de la base au catalyseur au cuivre est compris entre 1 et 2.

7